# EUROPEAN PATENT SPECIFICATION

(11) **EP 2 451 972 B1**
(45) Date of publication and mention of the grant of the patent: **04.05.2016**
(21) Application number: 10734406.1
(22) Date of filing: 09.07.2010
(51) Int. Cl.: C12Q 1/68

(54) **METHODS OF CLASSIFYING BIOLOGICAL SAMPLES FOR PREDICTING RESPONSE TO TYROSINE KINASE INHIBITOR TREATMENT**
METHODEN ZUR KLASSIFIZIERUNG VON BIOLOGISCHEN PROBEN ZUR VORHERSAGE DES ANSPRECHENS AUF DIE BEHANDLUNG MIT TYROSIN KINASE INHIBITOR
MÉTHODES POUR LA CLASSIFICATION DES ÉPREUVES BIOLOGIQUES POUR LA PRÉDICTION DE LA EFFICACITÉ DU TRAITEMENT AVEC TYROSINE KINASE INHIBITEUR

(30) Priority: 09.07.2009 US 224281 P
(43) Date of publication of application: 16.05.2012
(60) Divisional application: 16159330.6
(73) Proprietor: Abbott Molecular Inc., Des Plaines, IL 60018 (US)
(72) Inventor: COON, John, Oak Park Illinois 60302 (US); MORRISON, Larry, Lombard Illinois 60148 (US)
(74) Representative: Modiano, Micaela Nadia
(86) International application number: PCT/US2010/041512
(87) International publication number: WO 2011/006058

(56) References cited:
- WO-A1-2007/133516
- WO-A2-2005/117553
- WO-A2-2006/128195
- SOS M L ET AL: "Predicting drug susceptibility of non-small cell lung cancers based on genetic lesions" JOURNAL OF CLINICAL INVESTIGATION, AMERICAN SOCIETY FOR CLINICAL INVESTIGATION, US LNKD- DOI:10.1172/JCI37127, vol. 119, no. 6, 1 June 2009 (2009-06-01), pages 1727-1740, XP002555300 ISSN: 0021-9738 [retrieved on 2009-05-18]
- ENDOH HIDEKI ET AL: "PTEN and PIK3CA expression is associated with prolonged survival after gefitinib treatment in EGFR-mutated lung cancer patients." JOURNAL OF THORACIC ONCOLOGY : OFFICIAL PUBLICATION OF THE INTERNATIONAL ASSOCIATION FOR THE STUDY OF LUNG CANCER SEP 2006 LNKD- PUBMED:17409929, vol. 1, no. 7, September 2006 (2006-09), pages 629-634, XP002597655 ISSN: 1556-1380
- SHE QING-BAI ET AL: "Resistance to gefitinib in PTEN-null HER-overexpressing tumor cells can be overcome through restoration of PTEN function or pharmacologic modulation of constitutive phosphatidylinositol 3'-kinase/Akt pathway signaling." CLINICAL CANCER RESEARCH : AN OFFICIAL JOURNAL OF THE AMERICAN ASSOCIATION FOR CANCER RESEARCH 1 OCT 2003 LNKD- PUBMED:14555504, vol. 9, no. 12, 1 October 2003 (2003-10-01), pages 4340-4346, XP002597656 ISSN: 1078-0432
- MARSIT CARMEN J ET AL: "PTEN expression in non-small-cell lung cancer: evaluating its relation to tumor characteristics, allelic loss, and epigenetic alteration." HUMAN PATHOLOGY JUL 2005 LNKD- PUBMED:16084946, vol. 36, no. 7, July 2005 (2005-07), pages 768-776, XP002597657 ISSN: 0046-8177
- SOS MARTIN L ET AL: "PTEN loss contributes to erlotinib resistance in EGFR-mutant lung cancer by activation of Akt and EGFR." CANCER RESEARCH 15 APR 2009 LNKD- PUBMED:19351834, vol. 69, no. 8, 15 April 2009 (2009-04-15) , pages 3256-3261, XP002597658 ISSN: 1538-7445
- YAMAMOTO HIROMASA ET AL: "PIK3CA mutations and copy number gains in human lung cancers." CANCER RESEARCH 1 SEP 2008 LNKD- PUBMED:18757405, vol. 68, no. 17, 1 September 2008 (2008-09-01), pages 6913-6921, XP002597659 ISSN: 1538-7445
- DING LI ET AL: "Somatic mutations affect key pathways in lung adenocarcinoma." NATURE 23 OCT 2008 LNKD- PUBMED:18948947, vol. 455, no. 7216, 23 October 2008 (2008-10-23), pages 1069-1075, XP002597660 ISSN: 1476-4687
- MCDERMOTT ULTAN ET AL: "Identification of genotype-correlated sensitivity to selective kinase inhibitors by using high-throughput tumor cell line profiling." PROCEEDINGS OF THE NATIONAL ACADEMY OF SCIENCES OF THE UNITED STATES OF AMERICA 11 DEC 2007 LNKD- PUBMED:18077425, vol. 104, no. 50, 11 December 2007 (2007-12-11), pages 19936-19941, XP002597661 ISSN: 1091-6490
- M J FIDLER ET AL: "PTEN and PIK3CA gene copy numbers and poor outcomes in non-small cell lung cancer patients with gefitinib therapy", BRITISH JOURNAL OF CANCER, vol. 105, no. 12, 17 November 2011 (2011-11-17), pages 1920-1926, XP055215815, ISSN: 0007-0920, DOI: 10.1038/bjc.2011.494

## Description

### CROSS-REFERENCE TO RELATED APPLICATION

The present application claims priority to U.S. Provisional application number 61/224,281 filed July 9, 2009,

### BACKGROUND

EGFR mutation in exons 19 and 21 and high EGFR copy number have been associated with increased sensitivity to EGFR tyrosine kinase inhibitors (TKI's) in NSCLC patients, while KRAS mutations, MET expression, and EGFR T790M mutation have been linked to resistance. In preclinical models, EGFR downstream markers have been important in EGFR sensitivity or resistance. In this study we have examined the impact of abnormal PTEN and PIK3CA gene copy numbers on the outcomes of patients treated with gefitinib.

Relevant prior art literature includes:
Sos et al. (2009), J Clin Inv 119(6): 1727-1740, which shows a positive correlation between 3q26.2 gain and erlotinib activity;
Endoh et al. (2006), J Thor. Onc. 1 (7): 629-634, which shows that both higher PIK3CA and PTEN expression correlate with better response to gefitinib; She et al. (2003) Clin. Cancer Res., 9(12): 4340-4346, which shows that PTEN loss at location 10q23.3 contributes to resistance to gefitinib; Sos et al. (2009), Cancer Res., 69(8): 3256-3261, which shows that PTEN loss at location 10q23.3 contributes to resistance to erlotinib; WO 2007/133516 A1 (ABBOTT LAB [US]) (2007-11-22), which discloses a method to select a cancer patient who is predicted to benefit or not benefit from therapeutic administration of an EGFR inhibitor, comprising: a) detecting in a sample of tumor cells from a patient a level of a biomarker selected from the group consisting of: i) a level of amplification of the EGFR gene; ii) a level of polysomy of the EGFR gene; iii) a level of amplification of the HER2 gene; and iv) a level of polysomy of the HER2 gene.

Methods: Formalin-fixed paraffin embedded tissues and cell pellets from 81 gefitinibtreated NSCLC patients were analyzed by fluorescence in situ hybridization (FISH) with probes specific for EGFR, PTEN, PIK3CA, and centromeres (cen) 3, 7, and 10, and 72 specimens yielded results for all 6 probes. Patients were previously treated with at least one chemotherapy regimen or were considered ineligible for chemotherapy. FISH signals were enumerated in ≥40 cells per specimen to obtain copy numbers for each locus. Various classifiers were derived from the genomic copy numbers and a range of cutoff values were applied to classify patients by objective response (OR), progression free survival (PFS), and overall survival (OS). EGFR mutation status (exons 19 & 21) was obtained for 55 of the specimens.
Results: Loss of PTEN (≥20% of cells with <2 copies of PTEN) and low cen7 copy number (average cen7 copies/cell <4) were each significantly associated with lower OS, and high PIK3CA copy number (≥40% of cells with >2 copies of PIK3CA) and low EGFR copy number (<75% cells with >2 copies of EGFR) showed trends toward lower OS. Combined loss of PTEN and high PIK3CA copy number, or 3-way combinations of high PIK3CA copy number, loss of ten, and either low EGFR copy number or low cen7 copy number, were significantly associated with lower OS and lower PFS. Twenty two patients (31%) had tumors with high PIK3CA copy number, loss of PTEN, and low cen7 copy number, and these patients exhibited median OS of 132 d compared to 373 d in the other 50 patients (p<0.0001 Kaplan-Meier analysis, log rank test), median PFS of 62 d compared to 128 d (p=0.0007), and 0% OR compared to 22% (p=0,015, 2-tail Fischer's exact test). In the 37 patients that did not harbor an EGFR mutation, the classifier was still significant (median OS: 128 vs 380 d, p<0.0001; median PFS: 62 vs 102 d, p=0.019). In multivariate analysis, including PIK3CA, PTEN, and cen7 FISH status, gender, histology, and EGFR mutation status, all 3 FISH parameters remained significantly associated with OS.
Conclusions: These data indicate that gefitinib is relatively ineffective in tumors with relatively high PTEN loss and PIK3CA gain, and low levels of EGFR or cen7, in either the full study group or the subgroup without EGFR mutations. If confirmed in subsequent studies, alternative treatments, potentially including agents selected for combination with EGFR TKI's, should be considered for patients whose tumors have this profile.

### DESCRIPTION

The method of the invention is an clinical laboratory assay used to classify cancer or suspected cancer patient tissue samples into separate copy number profiles, indicative of response to tyrosine kinase inhibitors, such as gefitinib, which is a small molecule inhibitor of the EGFR receptor protein. The invention is the use of an in situ hybridization assay, preferably based on fluorescence in situ hybridization (FISH), using chromosomal hybridization probes to determine copy number at two, or three human chromosome loci: 10q23.3, 3q26.3 and chromosome 7, preferably either its centromere or at locus 7p12. Determination of the copy number at these loci in cells in a lung tissue sample can be used to classify the sample as having a copy number profile indicative of either resistance or response to a tyrosine kinase inhibitor. In the preferred embodiments, tissue sample determined as having relative loss of 10q23.3, relative gain of 3q26.3 and relative low copy number of chromosomes 7, compared to normal copy numbers at these loci, is classified as having a copy number profile of 10q23.3 loss, 3q26.3 gain and low copy number chromosome 7, which marks resistance to these inhibitors. Where the sample is classified as having any other copy number profile for these loci, the profile marks sensitivity or response to these inhibitors. In another preferred embodiment, the copy number profile is of the two loci 10q23.3 and 3q26.3. The invention is believed useful with all forms of tyrosine kinase inhibitors of genes in the EGFR pathway.
The invention is also notable because the copy number profile classification is independent of EGFR mutation status in the tissue sample.

The invention is also advantageous because the copy number profile was statistically significant over relatively wide ranges of percent assessable cells in the samples as having the particular chromosomal abnormality, ie 10q23.3 loss or 3q26.3 gain. For the 10q23.3 loss, the range of cells is about 13 to about 35% of the assessed cells showing relative loss, preferably about 20%. For the 3q26.3 gain, the range of cells is about 20 to about 62.5% of the assessed cells showing relative gain, preferably about 40%. The chromosome 7 copy number measurement showed robust statistical results for chromosome 7 copy numbers measured in the range of about 2.7 to about 5.1 of the assessed cells, preferably about 4 copies per cell.

The in situ hybridization chromosomal probes suitable for use in this invention include the EGFR and chromosome 7 centromere probes described in commonly assigned, co-pending U.S. application, "Diagnostic methods for determining treatment", L.E. Morrison and J.S. Coon, filed May 8, 2007, published as US 20070275403 A1. The in situ hybridization chromosomal probes suitable for use to determine copy number at the PIK3CA locu can be manufactured using the methods described in co-pending, commonly-assigned U.S. application, S.N. 12/268,797, "Prognostic Test for Early Stage Non Small Cell Lung Cancer", L.E. Morrison and J.S. Coon, filed November 11, 2008, herein "Morrison II", starting with clones containing human insert DNA containing nucleic acid sequences at the loci of PIK3CA. The Morrison II application also describes a suitable probe, designed to hybridize to the PTEN locus, for use herein. Probes useful herein are not limited to DNA based probes, but include peptide nucleic acid based probes. The in situ hybridization method used to determine the copy number profiles of this invention is described in our co-pending US applications. The copy numbers at each chromosome locus are determining preferably by standard FISH image analysis, either manual or using digital imaging software based methods.

## Claims

1. A method for classifying a biological sample with respect to treatment with an inhibitor of the tyrosine kinase activity of EGFR, the method comprising:
(a) contacting a biological sample obtained from a human patient with a set of chromosomal hybridization probes under conditions sufficient to enable hybridization of the probes to chromosomes in the sample wherein (i) one probe is designed to detect copy number of human Chromosome locus 10q23.3 in cells in the sample and (ii) one probe is designed to detect copy number of human Chromosome locus 3q26.3 in cells in the sample;
(b) determining copy number for each of human Chromosome locus 10q23.3 and human chromosome locus 3q26.3; and
(c) classifying the sample as resistant to treatment with an inhibitor of the tyrosine kinase activity of EGFR based on results of step (b), as having a copy number profile of 10q23.3 loss and 3q26.3 gain, relative to normal copy number for each.

2. The method of claim 1 further comprising in step (a) contacting the biological sample with (iii) a probe designed to detect copy number of human Chromosome 7 in cells in the sample; and in step (b) determining copy number for Chromosome 7; and wherein step (c) comprises classifying the sample as resistant to treatment with an inhibitor of the tyrosine kinase activity of EGFR based on results of step (b), as having a copy number profile of 10q23.3 loss, 3q26.3 gain, and chromosome 7 loss, relative to normal copy number for each.

3. The method of claim 2 wherein one of the probes is designed to hybridize to at least one nucleic acid sequence in human Chromosome 7 centromere.

4. The method of claim 2 wherein one of the probes is designed to hybridize to at least a one nucleic acid sequence present in a PTEN gene at locus 10q23.3, one of the probes is designed to hybridize to at least one nucleic acid sequence present in a PIK3CA gene at locus 3q26.3, and one of the probes is designed to hybridize to at least one nucleic acid sequence in human Chromosome 7 centromere.

5. The method of claim 2 wherein each of the probes is fluorescently labeled and are detectable simultaneously.

6. The method of claim 2 wherein one of the probes is designed to hybridize to at least one nucleic acid sequence in human EGFR gene at locus 7p12.

7. The method of claim 6 wherein one of the probes is designed to hybridize to at least a one nucleic acid sequence present in a PTEN gene at locus 10q23.3, and one of the probes is designed to hybridize to at least one nucleic acid sequence present in a PIK3CA gene at locus 3q26.3.

8. The method of claim 2 wherein the determining copy number step (c) comprises assessing at least 40 cells in the sample.

9. The method of claim 2 wherein copy number profile of 10q23.3 loss comprises determination that average Chromosome 7 copy number in assessable cells in the sample is less than 4.

10. The method of claim 1 or 2 wherein the biological sample is a lung biopsy sample.

11. The method of claim 1 or 2 wherein the biological sample is from a patient previously diagnosed as having non-small cell lung cancer.

12. The method of claim 1 or 2 wherein one of the probes is designed to hybridize to at least a one nucleic acid sequence present in a PTEN gene at locus 10q23.3.

13. The method of claim 1 wherein one of the probes is designed to hybridize to at least a one nucleic acid sequence present in a PTEN gene at locus 10q23.3, and one of the probes is designed to hybridize to at least one nucleic acid sequence present in a PIK3CA gene at locus 3q26.3.

14. The method of claim 1 or 2 wherein copy number profile of 10q23.3 loss comprises determination that ≥20% of cells have less than 2 copies of PTEN.

15. The method of claim 1 or 2 wherein copy number profile of 10q23.3 loss comprises determination that ≥40% of assessable cells in the sample have more than 2 copies of PIK3CA.

## Patentansprüche

1. Ein Verfahren zur Klassifizierung einer biologischen Probe im Hinblick auf die Behandlung mit einem Inhibitor der Tyrosinkinase-Aktivität von EGFR, wobei das Verfahren Folgendes umfasst:
(a) In-Kontakt-Bringen einer biologischen Probe, die von einem menschlichen Patienten gewonnen wurde, mit einem Satz chromosomaler Hybridisationssonden unter Bedingungen, die ausreichen, um die Hybridisierung der Sonden an Chromosomen in der Probe zu ermöglichen, worin (i) eine Sonde konstruiert ist, die Kopienzahl von humanem Chromosom-Lokus 10q23.3 in Zellen in der Probe zu bestimmen, und (ii) eine Sonde konstruiert ist, die Kopienzahl von humanem Chromosom-Lokus 3q26.3 in Zellen in der Probe zu bestimmen;
(b) Bestimmen der Kopienzahl sowohl für humanen Chromosom-Lokus 10q23.3 als auch für humanen Chromosom-Lokus 3q26.3; und
(c) Klassifizieren der Probe als resistent gegenüber Behandlung mit einem Inhibitor der Tyrosinkinase-Aktivität von EGFR, basierend auf den Ergebnissen von Schritt (b), da sie ein Kopienanzahl-Profil mit 10q23.3-Abnahme und 3q26.3-Zunahme relativ zur jeweiligen normalen Kopienanzahl hat.

2. Das Verfahren gemäß Anspruch 1, das weiter Folgendes umfasst: in Schritt (a): In-Kontakt-Bringen der biologischen Probe mit (iii) einer Sonde, konstruiert, um die Kopienanzahl von humanem Chromosom 7 in Zellen in der Probe zu bestimmen; und in Schritt (b): Bestimmen der Kopienanzahl für Chromosom 7; und worin Schritt (c) Folgendes umfasst: Klassifizieren der Probe als resistent gegenüber Behandlung mit einem Inhibitor der Tyrosinkinase-Aktivität von EGFR, basierend auf den Ergebnissen von Schritt (b), da sie ein Kopienanzahl-Profil mit 10q23.3-Abnahme und 3q26.3-Zunahme relativ zur jeweiligen normalen Kopienanzahl hat.

3. Das Verfahren gemäß Anspruch 2, worin eine der Sonden konstruiert ist, mit mindestens einer Nukleinsäuresequenz in humanem Chromosom 7-Zentromer zu hybridisieren.

4. Das Verfahren gemäß Anspruch 2, worin eine der Sonden konstruiert ist, mit mindestens einer Nukleinsäuresequenz zu hybridisieren, die in einem PTEN-Gen an Lokus 10q23.3 vorhanden ist, eine der Sonden konstruiert ist, mit mindestens einer Nukleinsäuresequenz zu hybridisieren, die in einem PIK3CA-Gen an Lokus 3q26.3 vorhanden ist, und eine der Sonden konstruiert ist, mit mindestens einer Nukleinsäuresequenz in humanem Chromosom 7-Zentromer zu hybridisieren.

5. Das Verfahren gemäß Anspruch 2, worin jede der Sonden fluoreszent markiert ist und sie gleichzeitig nachweisbar sind.

6. Das Verfahren gemäß Anspruch 2, worin eine der Sonden konstruiert ist, mit mindestens einer Nukleinsäuresequenz in humanem EGFR-Gen an Lokus 7p12 zu hybridisieren.

7. Das Verfahren gemäß Anspruch 6, worin eine der Sonden konstruiert ist, mit mindestens einer Nukleinsäuresequenz zu hybridisieren, die in einem PTEN-Gen an Lokus 10q23.3 vorhanden ist, und eine der Sonden konstruiert ist, mit mindestens einer Nukleinsäuresequenz zu hybridisieren, die in einem PIK3CA-Gen an Lokus 3q26.3 vorhanden ist.

8. Das Verfahren gemäß Anspruch 2, worin der Kopienanzahl-Bestimmungsschritt (c) die Bewertung von mindestens 40 Zellen in der Probe umfasst.

9. Das Verfahren gemäß Anspruch 2, worin das Kopienanzahl-Profil von 10q23.3-Abnahme die Bestimmung umfasst, dass die durchschnittliche Chromosom 7-Kopienanzahl in bewertbaren Zellen in der Probe kleiner als 4 ist.

10. Das Verfahren gemäß Anspruch 1 oder 2, worin die biologische Probe eine Lungenbiopsie-Probe ist.

11. Das Verfahren gemäß Anspruch 1 oder 2, worin die biologische Probe von einem Patienten stammt, bei dem zuvor nicht-kleinzelliger Lungenkrebs diagnostiziert wurde.

12. Das Verfahren gemäß Anspruch 1 oder 2, worin eine der Sonden konstruiert ist, mit mindestens einer Nukleinsäuresequenz zu hybridisieren, die in einem PTEN-Gen an Lokus 10q23.3 vorhanden ist.

13. Das Verfahren gemäß Anspruch 1, worin eine der Sonden konstruiert ist, mit mindestens einer Nukleinsäuresequenz zu hybridisieren, die in einem PTEN-Gen an Lokus 10q23.3 vorhanden ist, und eine der Sonden konstruiert ist, mit mindestens einer Nukleinsäuresequenz zu hybridisieren, die in einem PIK3CA-Gen an Lokus 3q26.3 vorhanden ist.

14. Das Verfahren gemäß Anspruch 1 oder 2, worin das Kopienanzahlprofil von 10q23.3-Abnahme die Bestimmung umfasst, dass ≥20% der Zellen weniger als 2 Kopien von PTEN haben.

15. Das Verfahren gemäß Anspruch 1 oder 2, worin das Kopienanzahlprofil von 10q23.3-Verlust die Bestimmung umfasst, dass ≥40% der bewertbaren Zellen in der Probe mehr als 2 Kopien von PIK3CA haben.

## Revendications

1. Procédé permettant de classer un échantillon biologique par rapport au traitement avec un inhibiteur de l'activité tyrosine kinase de l'EGFR, le procédé comprenant :
(a) la mise en contact d'un échantillon biologique obtenu d'un patient humain avec un ensemble de sondes d'hybridation chromosomique dans des conditions suffisantes pour permettre l'hybridation des sondes aux chromosomes dans l'échantillon, où (i) une sonde est conçue pour détecter le nombre de copies du locus de chromosome humain 10q23.3 dans les cellules dans l'échantillon et (ii) une sonde est conçue pour détecter le nombre de copies du locus de chromosome humain 3q26.3 dans les cellules dans l'échantillon ;
(b) la détermination du nombre de copies pour chacun du locus de chromosome humain 10q23.3 et du locus de chromosome humain 3q26.3 ; et
(c) le classement de l'échantillon comme résistant au traitement avec un inhibiteur de l'activité tyrosine kinase de l'EGFR en se basant sur les résultats de l'étape (b), comme ayant un profil de nombre de copies de perte de 10q23.3 et de gain de 3q26.3, par rapport au nombre de copies normal pour chacun.

2. Procédé selon la revendication 1 comprenant en outre : dans l'étape (a) la mise en contact de l'échantillon biologique avec (iii) une sonde conçue pour détecter le nombre de copies du chromosome 7 humain dans les cellules dans l'échantillon ; et dans l'étape (b) la détermination du nombre de copies pour le chromosome 7 ; et dans lequel l'étape (c) comprend le classement de l'échantillon comme résistant au traitement avec un inhibiteur de l'activité tyrosine kinase de l'EGFR en se basant sur les résultats de l'étape (b), comme ayant un profil de nombre de copies de perte de 10q23.3, de gain de 3q26.3, et de perte du chromosome 7, par rapport au nombre de copies normal pour chacun.

3. Procédé selon la revendication 2, dans lequel l'une des sondes est conçue pour s'hybrider à au moins une séquence d'acide nucléique dans le centromère du chromosome 7 humain.

4. Procédé selon la revendication 2, dans lequel l'une des sondes est conçue pour s'hybrider à au moins une séquence d'acide nucléique présente dans un gène PTEN au niveau du locus 10q23.3, l'une des sondes est conçue pour s'hybrider à au moins une séquence d'acide nucléique présente dans un gène PIK3CA au niveau du locus 3q26.3, et l'une des sondes est conçue pour s'hybrider à au moins une séquence d'acide nucléique dans le centromère du chromosome 7 humain.

5. Procédé selon la revendication 2, dans lequel chacune des sondes est marquée par fluorescence et est détectable simultanément.

6. Procédé selon la revendication 2, dans lequel l'une des sondes est conçue pour s'hybrider à au moins une séquence d'acide nucléique dans le gène EGFR humain au niveau du locus 7p12.

7. Procédé selon la revendication 6, dans lequel l'une des sondes est conçue pour s'hybrider à au moins une séquence d'acide nucléique présente dans un gène PTEN au niveau du locus 10q23.3, et l'une des sondes est conçue pour s'hybrider à au moins une séquence d'acide nucléique présente dans un gène PIK3CA au niveau du locus 3q26.3.

8. Procédé selon la revendication 2, dans lequel la détermination du nombre de copies de l'étape (c) comprend l'analyse d'au moins 40 cellules dans l'échantillon.

9. Procédé selon la revendication 2, dans lequel le profil de nombre de copies de perte de 10q23.3 comprend la détermination que le nombre de copies moyen du chromosome 7 dans les cellules analysables dans l'échantillon est inférieur à 4.

10. Procédé selon la revendication 1 ou 2, dans lequel l'échantillon biologique est un échantillon de biopsie pulmonaire.

11. Procédé selon la revendication 1 ou 2, dans lequel l'échantillon biologique provient d'un patient diagnostiqué au préalable comme souffrant d'un cancer du poumon non à petites cellules.

12. Procédé selon la revendication 1 ou 2, dans lequel l'une des sondes est conçue pour s'hybrider à au moins une séquence d'acide nucléique présente dans un gène PTEN au niveau du locus 10q23.3.

13. Procédé selon la revendication 1, dans lequel l'une des sondes est conçue pour s'hybrider à au moins une séquence d'acide nucléique présente dans un gène PTEN au niveau du locus 10q23.3, et l'une des sondes est conçue pour s'hybrider à au moins une séquence d'acide nucléique présente dans un gène PIK3CA au niveau du locus 3q26.3.

14. Procédé selon la revendication 1 ou 2, dans lequel le profil de nombre de copies de perte de 10q23.3 comprend la détermination que ≥ 20 % des cellules comportent moins de 2 copies de PTEN.

15. Procédé selon la revendication 1 ou 2, dans lequel le profil de nombre de copies de perte de 10q23.3 comprend la détermination que ≥ 40 % des cellules analysables dans l'échantillon comportent plus de 2 copies de PIK3CA.
